Europäisches Patentamt

European Patent Office

Office européen des brevets

(11) Publication number: **0 063 005**
**B1**

(12) **EUROPEAN PATENT SPECIFICATION**

(45) Date of publication of patent specification: **13.08.86**

(51) Int. Cl.⁴: **G 01 N 1/30, G 01 N 33/52**

(21) Application number: **82301716.5**

(22) Date of filing: **01.04.82**

(54) Silver staining method and kit.

(30) Priority: **02.04.81 US 250512**

(43) Date of publication of application:
**20.10.82 Bulletin 82/42**

(45) Publication of the grant of the patent:
**13.08.86 Bulletin 86/33**

(84) Designated Contracting States:
**BE CH DE FR GB IT LI NL SE**

(56) References cited:
**DE-A-3 001 264**

**CLINICAL CHEMISTRY, vol. 27, no. 2, 2
February 1981, Eastern Pennsylvania, USA, P.F.
GULYASSY et al. "Four methods for
determining albumin in azotemic sera
evaluated", pages 322-325**

**Chemical Abstracts, vol. 88, no. 4, 23 January
1978, Columbus, Ohio, USA, R.W. SMITH et al.,
"Staining natural rubber with silver nitrate for
electron microscopy", page 47, column 2,
abstract no. 24074v**

**Clin. Chim. Acta, 38, (1972), pages 465-467**

(73) Proprietor: **HEALTH PRODUCTS, INC.
P.O. Box 630
South Haven Michigan 49090 (US)**

(72) Inventor: **Adams, Lonnie Dean
6069 Paulson Road
Gobles Michigan (US)**
Inventor: **Sammons, David Winston
703 South Rose Street
Kalamazoo Michigan (US)**

(74) Representative: **Perry, Robert Edward et al
GILL JENNINGS & EVERY 53-64 Chancery Lane
London WC2A 1HN (GB)**

Courier Press, Leamington Spa, England.

## Description

The present invention involves a novel silver staining method and a kit useful in practicing said method.

There are a number of known methods useful in staining proteins which utilize silver. For example, L. Kerenyi et. al., Clin, Chim, Acta 38, 465—467 (1972), describe a method for demonstrating proteins in electrophoretic, immunoelectrophoretic and immunodiffusion preparations whereby the preparations are treated with potassium ferrocyanide which is transformed during development into silver ferrocyanide then into colloidal silver grains. The physical developer contains anhydrous sodium carbonate, ammonium nitrate, silver nitrate, tungstosilicic acid and formalin, and the protein in the preparations stain dark-brown with a pale gray background.

R. C. Switzer et. al., Anal. Biochem, 98, 231—237 (1979), and C. R. Merril et. al., Proc. Nat'l. Acad. Sci. USA, 76, No. 9, 4335—4339 (1979), describe a silver stain technique for detecting proteins and peptides in polyacrylamide gel which is a modification of de Olmos' neural, cupric-silver stain. The procedure consists of ten steps and utilizes an aqueous solution of silver nitrate and cupric nitrate and involves treatment with a diammine solution which is known to sometimes form an explosive silver amide complex. The proteins stain as dark spots on a darkened background.

B. A. Oakley et. al., Anal. Biochem. 105, 361—363 (1980), simplified the procedure of Switzer et. al., by reducing the number of steps involved to six and also reducing the amount of silver required without diminishing the sensitivity of the technique. However, the manner in which the proteins stain was not changed, i.e., dark stain on a darkened background.

A further modification of the Switzer et. al. procedure was made by R. C. Allen, Electrophoresis I. 32—37 (1980), who increased the sodium to ammonium ion ratio which resulted in increased silver deposition.

C. R. Merril et. al., Anal. Biochem. 110, 201—207 (1981) modified and simplified the above procedure of Kernyi et. al., adapting it to acrylamide gels.

D. Goldman et al., Science 211 (1981) 1437—1438, describe a silver stain procedure for proteins separated by two-dimensional gel electrophoresis, which requires treatment with potassium dichromate and nitric acid prior to staining with silver nitrate, followed by washing then immersion in an image developer containing formalin and sodium carbonate. There is no indication of colour development with this stain procedure.

All the silver stain techniques described above, although perhaps sensitive, only stain proteins in varying shades of brown or black.

D. Goldman et al., Clin, Chem. 26(9), (1980) 1317—1322, report that, when using a procedure essentially the same as that of Merril et al., Proc. Nat. Acad. Sci, supra, and Switzer et al., Anal. Biochem., supra, proteins from samples of cerebral spinal fluid were stained shades of yellow, red and blue.

According to the present invention, a method for staining a substance capable of binding silver and which is supported in a matrix, which comprises equilibrating the substance in an aqueous silver salt solution; rinsing the substance in water; treating the substance with a basis reducing solution; and immersing the substance in a first, and then in a second, aqueous carbonate or sulfate salt solution.

A method of the present invention is highly sensitive, making is possible to detect picogram quantities of substances which are capable of binding silver. The novel method also makes it possible to stain substances, and especially proteins, in varying shades of blue, green, red and yellow.

The substances to be stained bind silver according to their unique composition, thereby resulting in a characteristic colour. The colour adds a new dimension to the separation and identification of substances by electrophoreses by aiding, for example, the resolution of overlapping proteins, particularly, and identifying families of proteins, i.e., proteins comprised of the same protein subunits but which may be conjugated to other chemical entities such as saccharides, by altering the overall net charge or character of the proteins. The method of the present invention thus facilitates the identification of substances which are diagnostic markers and/or primary lesions of most genetic diseases, including major diseases such as diabetes, atherosclerosis and cancer. In addition, pathological changes in tissues can be correlated with changes in certain substances, particularly proteins. The method of the present invention provides a means for enhancing understanding of normal and disease conditions.

According to a further aspect of the present invention, a kit, suitable for use in a method of staining a substance capable of binding silver, comprises five containers each containing a different one of five reagents which are a water-soluble silver salt, a base solution, a borohydride, an aldehyde solution and a water-soluble carbonate or sulfate salt.

Such a kit may comprise up to 6, preferably 1.85 to 2 g silver salt; from 100 to 150 ml base; from 150 to 200 mg borohydride reducing agent; from 12 to 20 ml aldehyde; and from 20 to 50 g carbonate or sulfate salt.

Substances which are capable of binding silver and which can be stained by the method of the present invention includes proteins, polypeptides, amino-acids, nucleic acids and polymers thereof, lipids, carbohydrates (including starches and sugars of various classes, e.g. oligosaccharides or polysaccharides such as mucoitin sulfate), lipoproteins, glucoproteins, nucleoproteins (including ribonucleic and deoxyribonucleic protein complexes), mucopolysaccharides such as chondroitin, proteoglycans, mucolipids such as ganglioside, mucoproteins, and glycolipids.

It is advantageous, when staining a "matrix preparation", i.e. a sample of substance supported in a matrix, by the method of the present invention, that said matrix preparation be washed thoroughly to remove any components which may interfere with the uptake of silver by the substance to be stained. The matrix preparation should be washed repeatedly, for example, in a lower alkanol, such as methanol or ethanol containing a mild acid, such as, trichloroacetic acid or acetic acid. Generally, for a 1.5 mm thick gel, washing for 2 to 12 hours is adequate using for each milliliter (ml) of matrix 5.5 ml of solution. Of course, less time is required for thinner gels. This washing procedure is commonly referred to as fixation/washing.

The present novel staining method can be used to stain virtually any substance which is capable of binding silver and typically is useful in staining such substances which have been separated using one or two dimensional electrophoresis techniques. The matrix in which the substance to be stained is supported can be comprised of any materials which are commonly used in electrophoretic procedures. For example, the matrix may consist of filter paper, cellulose acetate, starch gel, agarose, Sephadex beads, or polyacrylamide, and may vary in thickness from an ultrathin matrix of about 80 μm to 3 mm. It may be desirable to attach the matrix on a nylon mesh, a glass plate, or a plastic sheet for additional support. As is known from standard electrophoretic techniques, the density and pore size of the matrix may also vary. A preferred matrix composition is polyacrylamide, varying from 80 μm to 1.5 mm in thickness with 1.5 mm being the most preferred thickness.

We have found that the present method is especialy useful for staining substances, and, in particular, proteins that have been separated by two dimensional electrophoreses on polyacrylamide gels. The method of the present invention is particularly useful for staining substances separated using the ISO—DALT system of Anderson and Anderson, Anal. Biochem. 85, 331—340 and 341—354 (1978). When staining substances separated by the ISO—DALT system, sequential washing with agitation of the gel preparation in solutions of ethanol or methanol of concentrations varying from about 50% to 40% to 25% to 10% containing a suitable mild acid, such as acetic acid, at a concentration of 10% in the first three solutions and 0.5% in the final solution, is generally adequate to remove any sodium dodecyl sulfate contained in the gel.

With the method of the present invention we have been able to detect picogram quantities of substances and obtain matrix preparations which stain in varying shades of blue, green, yellow and red. The background of the matrix preparation stains in light shades of yellow to orange upon which the brightly colored substances are detected easily. The intensity of the color will vary somewhat with the thickness of the matrix as well as with the concentration of the substance to be stained. For best results a matrix of 1.5 mm thickness of 10% to 20% polyacrylamide gel is used.

The novel silver stain procedure described and claimed herein is highly sensitive, gives high resolution of substances, is reproducible, is easy to use, and is efficient in that matrix preparations can be stained batch-wise. Additionally the entire procedure is carried out at room temperature. i.e., about 18° to 27°C with standard room lighting, there being no requirement of elevated temperatures or special light control.

Suitable silver salts which may be used in the present invention are those which will dissociate in water to give free siver ion and which will not interact with the reducing agents employed in the method. Aqueous solutions of silver nitrate or silver acetate are particularly suitable for use in the present invention. The silver salt is dissolved in water, generally distilled water, at a concentration of from about 0.5 to 6 grams of silver salt per liter of water. The lower concentration of silver salt, i.e., 1 gram/liter results in reduced sensitivity and intensity and the backgroud of the matrix stains lighter than at the higher concentrations of silver salt. When ultrathin matrices are used concentrations of silver salt varying from about 3 to 6 grams of salt per liter of water are preferred. A preferred concentration of silver salt for thicker gels, i.e., 0.75 to 1.5 mm, is about 1.5 to 2.0 grams/liter, and the more preferred concentration is about 1.90 grams/liter. The matrix preparation is equilibrated in the silver salt solution for a minimum of about two hours using for each 1 ml of matrix about 3 ml of solution. Of course the thicker matrix preparations require more time to equilibrate than do the thinner matrix preparations.

Following equilibration in the silver salt solution the matrix preparation is washed briefly in water to remove any surface silver, then the preparation is subjected to a reducing solution. The reducing solution may be sprayed onto the matrix preparation, particularly when thin matrix preparations are being stained, or the matrix preparation may be immersed in the reducing solution. The reducing solution consists of a base such as aqueous potassium hydroxide or sodium hydroxide or sodium or potassium carbonate and an aldehyde, such as, formaldehyde, acetaldehyde, n-butyraldehyde, or glutaraldehyde with or without addition of a metal borohydride reducing agent such as sodium borohydride or potassium borohydride. The concentration of base may vary from about 0.5 to 1.0 N with 0.75 N base, preferably sodium hydroxide, being the most suitable. The preferred reducing agent is formaldehyde and generally a 37% solution is employed. Generally the reducing solution will comprise about 1 to 5 ml of 37% formalin per about each 400 ml of aqueous base. When a metal borohydride reducing agent is used generally about 25 to 50 mg per about each 400 ml of aqueous base is employed. A preferred reducing solution consists of per each 390 ml of 0.75 N NaOH, 3 ml of 37% formalin and 35 mg of NaBH$_4$ in 7 ml of 0.75 N sodium hydroxide. For each ml of matrix preparation one should use about 5.5 ml of reducing solution. It is recommended that in preparing the reducing solution as well as other aqueous solutions described herein one use distilled deionized and degassed water. The reduction generally requires about 5 to 15 minutes, and when the matrix preparation is submersed in the reducing solution agitation at about 40 cycles per minute is recommended. The time required for reduction varies with the thickness of the gels with time required increasing as the matrix thickness increases. The reaction time could be shortened if the temperature is raised.

When sodium carbonate as a 3% solution is used as the base in the reducing solution the background matrix has a tendency to stain very dark which may obscure the color staining pattern. The dark background can be overcome in several ways. For example, the length of time the matrix preparation is subjected to the reducing solution may be shortened followed by treatment with a mild acid, such as acetic acid. Also by placing the matrix preparation in the sodium carbonate solution then carefully titrating the aldehyde, preferably a formaldehyde, the dark background staining is avoided. Also, by careful addition of an oxidizing agent such as sodium sulfite to prevent rapid and extensive surface reaction the dark background staining is prevented. Although sodium carbonate is useful as a base in the reducing solution, it is not preferred in view of the additional preparative steps required to give a satisfactory product.

Generally the components of the reducing solution are mixed together and the matrix preparation previously equilibrated in the silver salt solution is brought into contact with the resultant solution. The reduction step may also be achieved by first contacting the matrix preparation with a suitable base solution followed by the addition of the aldehyde and optionally the borohydride reducing agent, however, the reverse addition of components is less desirable.

Following reduction the matrix preparation is preferably immersed for about one hour in an 0.075 to 1.5 percent (%) anhydrous sodium or potassium carbonate solution or a sodium or potassium sulfate solution having a pH of 11 or greater followed by immersion in a second sodium or potassium carbonate or sulfate solution which may vary in concentration from about 0.15 to 3.0%, preferably about 0.75%. The matrix preparation may be stored in the second carbonate salt solution which also has a pH of 11 or greater. Sodium carbonate is preferred for this step. Optimal color develops in about 6 hours giving, in addition to varying shades of black and brown, varying shades of blue, green, yellow, and red. The stained preparation may be photographed by standard well known techniques. If one wishes, for example, in those instances where color photography is not practicable, the color development may be avoided by substituting ammonium carbonate or ammonium sulfate for sodium carbonate or sulfate in the final two wash solutions. The quantity of ammonium carbonate or ammonium sulfate employed may vary from 1% to 3% with 1.5% being particularly suitable. The resultant matrix preparation will be stained in varying shades of brown, black and yellow.

As indicated hereinabove standard electrophoretic procedures may be used to separate the substances to be stained in either one dimension or two dimensions. When such substances have been separated using standard two dimensional electrophoresis on polyacrylamide gel, it is very important to remove the SDS during the fixation/ washing step. Also it is recommended that reagents of high quality by used. Additionally, it is recommended that for best results a freshly prepared solution of 2-mercaptoethanol be made up for reducing agent, and that sodium hydroxide and phosphoric acid by used for cathodic and anodic solutions respectively.

When preparing the sample of substance to be stained the concentration for each sample will have to be adjusted individually. Generally, we find that samples should be diluted 10 to 50 times of those used for staining by conventional Coomassie Blue procedures.

A preferred general procedure is the following.

General Procedure

Tissue samples or samples of substances to be stained are prepared and run on first and second dimension gels according to the procedure described by Anderson and Anderson, ibid. Second dimension gels are fixed in 50% ethanol/10% acetic acid for two or more hours with one change of fixative. The gels are then washed two times in 25% ethanol/10% acetic acid and two times in 10% ethanol/0.5% acetic acid (one hour each) to remove any remaining sodium dodecyl sulfate (SDS). Next the gels are soaked two or more hours in 1.9 g/l AgNO$_3$ in water. Gels are removed from the silver solution and processed singly or in groups of up to five or more. Single gels are rinsed in deionized water and then placed in a rectangular glass dish 28 × 18 × 4 cm. To this dish is added reducing solution which consists of 390 ml 0.75N NaOH, 3 ml of 37% formaldehyde, and 7 ml of 5 mg/ml NaBH$_4$ in aqueous 0.75N NaOH. The dish is placed on a shaker for 10 minutes, then the reducing solution is poured off and replaced with an equal volume of 0.75% Na$_2$CO$_3$. After an hour the Na$_2$CO$_3$ solution is replaced with fresh 0.75% Na$_2$CO$_3$ solution in which the gel is stored. For more than one gel, appropriately larger volumes of reducing solution and Na$_2$CO$_3$ solution are used.

Example 1

In order to determine the sensitivity of the staining method and to determine the effect, if any, of concentration of protein on color, development mixtures of proteins of known concentrations were electrophoresed using the general procedure of the two dimensional ISO—DALT technique then stained by the General Procedure set forth above. Seven mixtures consisting of creatine kinase, myokinase, α-amylase, lactate dehydrogenase I (LDH-I) and glutamate-pyruvate transaminase (GPT) are prepared. The concentration of each of the proteins in any one mixture is the same, and the concentrations are 1 ng, 2 ng, 10 ng, 50 ng, 200 ng, 1 µg or 5 µg.

All five proteins can be seen clearly at the 5 µg, 1 µg and 200 ng concentrations, however, α-amylase and GPT are faint at the 200 ng concentration indicating that the sensitivity of the staining procedure varies somewhat with the protein. LDH—I can also be seen at the 50 ng, 10 ng and 2 ng concentrations. No protein at the 1 ng concentration is detected. At the 2 ng level LDH—I appears as 3 spots very close together which indicates each spot represents less than 1 ng of protein.

The proteins stained the following colors: Creatine kinase, blue-green; myokinase, yellow; alpha amylase, a red shade; LDH—I, blue-gray; and GPT, blue-gray. At low concentrations the hue of the proteins was unchanged as a function of concentration. However, at higher saturated concentrations of protein mixed colors result. For example, at concentrations of 5 µg and 1 µg LDH—I spots have a red center with a blue margin. Also, at a concentration of 5 µg the myokinase spot has a black center with a yellow margin. The mixed color is believed to be due to an absence of uniform silver deposition throughout the protein spot.

### Example 2

The significant reagents for the performance of the staining procedure are assembled into a mercantile kit, specifically, a kit for marketing to qualified individuals to perform the staining procedure. The kit comprises as basic components at least four containers, one of each having therein appropriate amounts of a suitable silver salt; a suitable base; a suitable aldehyde reducing agent; or an appropriate amount of sodium carbonate. The kit may have an additional container having therein an appropriate amount of a metal borohydride reducing reagent.

A preferred kit composition is one designed to stain 6 gels which are 1.5 mm thick and are 18 cm × 20 cm wherein one container has therein 1.85 to 2.0 grams of silver nitrate; another container has therein 120 ml of 50% sodium hydroxide; another container has therein 175 mg of sodium borohydride; another container has therein 16 ml of 37% formalin; and two additional containers have therein 22 grams of sodium carbonate.

**Claims**

1. A method for staining a substance capable of binding silver and which is supported in a matrix, including the use of an aqueous silver salt solution, characterised in that the matrix-bound substance is subjected to the following steps, sequentially:

(1) equilibration in an aqueous silver salt solution;

(2) rinsing in water;

(3) treatment with a basic reducing solution; and

(4) immersion in a first, and then in a second, aqueous carbonate or sulphate salt solution.

2. A method according to any preceding claim, wherein the substance, prior to the equilibration, is fixed and washed in an aqueous lower alkanol solution containing a mild acid.

3. A method according to claim 1 or claim 2, wherein the matrix is polyacrylamide gel and the silver salt is silver nitrate.

4. A method according to any preceding claim, wherein the reducing solution comprises a base selected from aqueous potassium hydroxide, aqueous sodium hydroxide and formaldehyde.

5. A method according to claim 4, wherein the reducing solution additionally comprises sodium borohydride.

6. A method according to any preceding claim, wherein the substance is equilibrated for at least two hours, is treated with the reducing solution for from 5 to 15 minutes, and then immersed in the first aqueous carbonate or sulfate salt solution for about one hour and in the second aqueous carbonate or sulfate salt solution for about six hours.

7. A method according to any preceding claim, wherein the carbonate salt is sodium carbonate.

8. A method according to any preceding claim, wherein the substance is a protein, a polypeptide or a protein-containing substance.

9. A method according to claim 8, wherein the substance is equilibrated for about two hours in an aqueous solution containing from 0.5 to 6 g/l silver nitrate; is treated for from 5 to 15 minutes with an aqueous reducing solution comprising sodium hydroxide, formaldehyde and sodium borohydride in amounts corresponding to 1 to 5 ml of 37% formalin and 25 to 50 mg of sodium borohydride per 400 ml of 0.5 to 1.0 N aqueous sodium hydroxide; and is then immersed, firstly in a 0.075 to 1.5% aqueous sodium carbonate solution for about one hour, and secondly in a 0.15 to 3% aqueous sodium carbonate solution for about six hours.

10. A kit, suitable for use in a method according to any preceding claim, which comprises five containers each containing a different one of five reagents which are a water-soluble silver salt, a base solution, a borohydride, an aldehyde solution and a water-soluble carbonate or sulfate salt.

**Patentansprüche**

1. Verfahren zum Färben einer Substanz, die zur Bindung von Silber befähigt ist und in einer Matrix getragen wird, welches die Verwendung einer wässrigen Silbersalzlösung umfaßt, dadurch gekennzeichnet, daß die Matrix-gebundene Substanz nacheinander der folgenden Schritten unterworfen wird:

(1) Äquilibrierung einer wässrigen Sibersalzlösung;

(2) Spülen in Wasser;

(3) Behandeln mit einer basischen reduzierenden Lösung; und

(4) Eintauchen in eine erste und dann in eine zweite wässrige Carbonat- oder Sulfat-Salzlösung.

2. Verfahren nach irgendeinem vorangehenden Anspruch, worin die Substanz vor der Äquilibrierung fixiert und in einer wässrigen Lösung eines niederen Alkanols, die eine milde Säure enthält, gewaschen wird.

3. Verfahren nach Anspruch 1 oder Anspruch 2, worin die Matrix ein Polyacrylamid-Gel und das Silbersalz Silbernitrat ist.

4. Verfahren nach irgendeinem vorangehenden Anspruch, worin die reduzierende Lösung eine Base, ausgewählt unter wässrigem Kaliumhydroxid, wässrigem Natriumhydroxid und Formaldehyd umfaßt.

5. Verfahren nach Anspruch 4, worin die reduzierende Lösung zusätzlich Natriumborhydrid umfaßt.

6. Verfahren nach irgendeinem vorangehenden Anspruch, worin die Substanz mindestens 2 Stunden äquilibriert, 5—15 Minuten mit der reduzierenden Lösung behandelt und dann etwa 1 Stunde in die erste wässrige Carbonat- oder Sulfat- Salzlösung und etwa 6 Stunden in die zweite wässrige Carbonat- oder Sulfat-Salzlösung getaucht wird.

7. Verfahren nach irgendeinem vorangehenden Anspruch, worin das Carbonatsatz Natriumcarbonat ist.

8. Verfahren nach irgendeinem vorangehenden Anspruch, worin die Substanz ein Protein, ein Polypeptid oder eine proteinhaltige Substanz ist.

9. Verfahren nach Anspruch 8, worin die Substanz etwa 2 Stunden in einer wässrigen Lösung, die 0,5—6 g/l Silbernitrat enthält, äquilibriert wird, 5—15 Minuten mit einer wässrigen reduzierenden Lösung, die Natriumhydroxid, Formaldehyd und Natriumborhydrid in Mengen, entsprechend 1—5 ml 37% Formalin und 25—50 mg Natriumborhydrid pro 400 ml wässrigem 0,5 bis 1.0 N Natriumhydroxid, umfaßt, behandelt wird und dann zunächst etwa 1 Stunde in eine wässrige 0,075 bis 1,5% Natriumcarbonatlösung und danach etwa 6 Stunden in eine wässrige 0,15 bis 3% Natriumcarbonatlösung getaucht wird.

10. Reagenzsatz zur Durchführung des Verfahrens nach irgendeinem vorangehenden Anspruch, umfassend 5 Behälter mit einem Gehalt an jeweils einem verschiedenen von 5 Reagenzien, welche sind: ein wasserlösliches Silbersalz, eine Basenlösung, ein Borhydrid, eine Aldehydlösung und ein wasserlösliches Carbonat- oder Sulfatsalz.

**Revendications**

1. Procédé pour la coloration d'une substance capable de fixer l'argent et qui est supportée dans une matrice, comprenant l'utilisation d'une solution aqueuse d'un sel d'argent, caractérisé en ce que la substance fixée sur la matrice est soumise aux étapes successives suivantes:

(1) équilibrage dans une solution aqueuse de sel d'argent;

(2) rinçage dans l'eau;

(3) traitement par une solution réductrice basique; et

(4) immersion dans une première, puis une seconde solution aqueuse de carbonate ou de sulfate.

2. Procédé selon la revendication 1, dans lequel la substance est fixée avant l'équilibrage et lavée dans une solution aqueuse d'un alcohol inférieur contenant un acide faible.

3. Procédé selon la revendication 1 ou 2, dans lequel la matrice est un gel de polyacrylamide et le sel d'argent est le nitrate d'argent.

4. Procédé selon l'une quelconque des revendications précédentes, dans lequel la solution réductrice comprend une base choisie parmi l'hydroxyde de potassium aqueux et l'hydroxyde de sodium aqueux et du formaldéhyde.

5. Procédé selon la revendication 4, dans lequel la solution réductrice contient en outre du borohydrure de sodium.

6. Procédé selon l'une quelconque des revendications précédentes, dans lequel la substance est équilibrée pendant au moins 2 heurs, traitée par la solution réductrice pendant 5 à 15 minutes et ensuite plongée dans la première solution aqueuse de carbonate ou de sulfate pendant environ 1 heure et dans la seconde solution aqueuse de carbonate ou de sulfate pendant environ 6 heurs.

7. Procédé selon l'une quelconque des revendications précédentes, dans lequel le carbonate est le carbonate de sodium.

8. Procédé selon l'une quelconque des revendications précédentes, dans lequel la substance est un protéine, un polypeptide ou une substance contenant une protéine.

9. Procédé selon la revendication 8, dans lequel la substance est équilibrée pendant environ 2 heures dans une solution aqueuse contenant de 0,5 à 6 g/l de nitrate d'argent; traitée pendant 5 à 15 minutes avec une solution réductrice aqueuse comprenant de l'hydroxyde de sodium, du formaldéhyde et du borohydrure de sodium en quantités correspondnat à 1 à 5 ml de formaline à 37% et 25 à 50 mg de borohydrure de sodium par 400 ml d'hydroxyde de sodium aqueux 0,5 à 1,0 N; et ensuite plongée d'abord dans une solution aqueuse de carbonate de sodium à 0,075—1,5 % pendant environ 1 heure et ensuite dans une solution aqueuse à 0,15—3% de carbonate de sodium pendant environ 6 heures.

10. Trousse d'essai appropriée pour l'utilisation dans un procédé selon l'une quelconque des revendications précédentes, qui comprend cinq récipients contenant chacun un réactif différent choisi parmi les cinq suivants: un sel d'argent soluble dans l'eau, une solution d'une base, un borohydrure, une solution d'aldéhyde et un carbonate ou sulfate soluble dans l'eau.